# FASCICULE DE BREVET EUROPEEN

(11) **EP 2 211 660 B1**
(45) Date de publication et mention de la délivrance du brevet: **20.02.2019**
(21) Numéro de dépôt: 08871362.3
(22) Date de dépôt: 24.10.2008
(51) Int. Cl.: A45D 34/02, A45D 34/00, A45D 34/04, A61L 9/12, G09F 5/00

(54) **DISPOSITIF DE CONSERVATION ET DE RESTITUTION D'UNE FRAGRANCE, ET ENSEMBLE DE TELS DISPOSITIFS**
VORRICHTUNG ZUR LAGERUNG UND AUSGABE EINES DUFTSTOFFS UND SATZ VON SOLCHEN VORRICHTUNGEN
DEVICE FOR STORING AND RELEASING A FRAGRANCE, AND SET OF SUCH DEVICES

(30) Priorité: 25.10.2007 FR 0758572
(43) Date de publication de la demande: 04.08.2010
(73) Titulaire: Chanel Parfums Beauté, 92200 Neuilly-sur-Seine (FR)
(72) Inventeur: MALLOCHET Cédric, F-95870 Bezons (FR); SHELDRAKE, Christopher, 92500 Rueil Malmaison (FR); MADIOT, Gaelle, F-92250 La Garenne Colombes (FR)
(74) Mandataire: Santarelli
(86) Numéro de dépôt international: PCT/FR2008/001501
(87) Numéro de publication internationale: WO 2009/092874

(56) Documents cités:
- EP-A- 0 297 959
- WO-A-02/083417
- CH-A- 385 436
- GB-A- 2 389 790
- US-A- 5 031 764
- US-A1- 2002 094 225
- US-A1- 2005 220 664

## Description

L'invention concerne un dispositif de conservation et de restitution d'une fragrance, notamment un parfum.

Ainsi qu'on le sait, la découverte de fragrances, en vue notamment de la finalisation d'un choix de consommation, implique de tester plusieurs fragrances pour finir par arrêter son choix sur une fragrance donnée.

Il est courant, à cet effet, d'utiliser des bandes de papier absorbant (dans le domaine des parfums, on parle souvent de « touche » de papier). Plus précisément, la démonstratrice présente successivement à son interlocuteur (ou interlocutrice) de telles bandes après y avoir vaporisé une petite quantité de l'une ou l'autre des fragrances entre lesquelles un choix est à faire.

Mais une telle démarche a l'inconvénient de nécessiter, pour chaque fragrance, la vaporisation d'une quantité souvent supérieure à celle que peut absorber la bande de papier, ce qui conduit à des pertes de produit (produit vaporisé à côté de la bande), à des risques de formation de gouttes disgracieuses, à un chargement incontrôlé de chaque bande (certaines peuvent être saturées tandis que d'autres sont à peine chargées), ce qui rend difficile de comparer la puissance des diverses fragrances, ainsi qu'à un chargement progressif de l'environnement avec l'ensemble des fragrances testées, ce qui fausse progressivement l'évaluation des nouvelles fragrances proposées.

Il faut noter que le fait que les bandes ne s'utilisent qu'une fois a pour effet que le produit restant sur la bande est jeté alors qu'il n'a pas complètement été évaporé.

En outre, l'usage de simples bandes de papier pour la présentation de parfums ne permet pas toujours de mettre l'interlocuteur ou l'interlocutrice dans l'état d'esprit adéquat pour évaluer la sophistication des fragrances proposées, notamment dans le cas de parfums de luxe.

Un usage particulier de telles bandes a été proposé en combinaison avec une cloche posée sur une surface plane et au fond de laquelle on fixe temporairement la bande de papier. Cela permet que la bande de papier dégage la fragrance dont elle est chargée dans tout le volume de la cloche, ce qui permet à l'interlocuteur ou interlocutrice d'inspirer profondément la fragrance pour en apprécier les qualités. Mais une telle solution consomme un espace important sur la surface plane en question, et n'évite pas les mélanges après la manipulation de plusieurs cloches.

On a aussi proposé de disposer des tissus imprégnés dans des enceintes que l'on ouvre successivement pour y prélever et manipuler les tissus et évaluer les fragrances d'imprégnation, mais cela peut avoir pour inconvénient que la démonstratrice se sature les doigts avec les fragrances successives.

On connaît déjà, d'après le document US - 2005/0220664, un dispositif de distribution d'une substance aromatique comportant une enceinte contenant en pratique la substance à l'état liquide et dans lequel plonge un élément vertical dans lequel la substance se charge par capillarité puis s'évapore vers l'extérieur, de manière contrôlée ; toutefois, un tel dispositif a une structure bien différente de celle du dispositif de l'invention, ne convenant notamment pas à la présentation d'un parfum à une consommatrice.

Il existe donc un besoin de pouvoir présenter des fragrances, telles que des parfums, en minimisant les pertes de produit (ne servant pas à évaluer le produit en question) et les risques de chargement intempestif de l'atmosphère environnante, tout en garantissant une certaine constance dans la présentation d'une fragrance donnée et tout en permettant à la démonstratrice de conférer un certain cérémonial à la démonstration d'une pluralité de fragrances ; en pratique la constance précitée correspond à un objectif d'apporter une restitution pure et fidèle de la fragrance concerné, ce qui implique à la fois une bonne conservation de la fragrance et, au moment opportun, une bonne restitution, reproductible, de celle-ci.

L'invention propose ainsi un dispositif de conservation et de restitution d'une fragrance donnée comportant les caractéristiques de la revendication 1.

On appréciera que, selon l'invention, la fragrance est ainsi stockée, dans son enceinte de confinement, principalement au sein de la portion poreuse de la tige, ce qui minimise les risques de perte de produit ou de formation de goutte ; il n'y a plus de vaporisation puisque le chargement de la portion poreuse de la tige peut se faire par simple trempage dans la fragrance liquide, à l'extérieur de l'enceinte de confinement, donc avant engagement de la tige poreuse dans son enceinte de confinement, et le fait que, lorsque la tige est dans son enceinte de confinement, cette tige (en tout cas sa portion poreuse) n'est pas en contact avec de la fragrance à l'état liquide garantit qu'il n'y a pas de formation de goutte. Le fait que la tige ait une tête qui obture le col de l'enceinte de confinement minimise les risques d'échappement de la fragrance emmagasinée dans cette enceinte vers l'extérieur, de sorte qu'il n'y a que peu (voire pas du tout) de chargement de l'atmosphère en cette fragrance ; en outre, puisque la fragrance emmagasinée dans l'enceinte de confinement ne peut pas facilement s'échapper, il en découle que, lorsque la tige est à nouveau engagée dans cette enceinte, la fragrance stockée dans la portion poreuse n'est que peu sollicitée pour s'évaporer dans cette enceinte, de sorte que cette tige présente la fragrance dans des conditions sensiblement identiques au fur et à mesure des extractions successives hors de l'enceinte de confinement.

Il faut noter que, puisque le stockage de la fragrance se fait principalement au sein de la portion poreuse, le transport de la tige de présentation engagée dans son enceinte de confinement permet un transport de la fragrance indépendamment de tout transport de liquide, ce qui a notamment pour avantage d'éviter tout risque de fuite.

La tête de préhension est avantageusement conçue en sorte d'éviter une évaporation de la fragrance au travers de celle-ci. A cet effet, cette tête est de préférence au moins en partie réalisée en un matériau non poreux ; c'est ainsi par exemple qu'elle est avantageusement en un matériau non poreux, ou au contraire en un matériau poreux (par exemple en un une seule pièce avec la tige) recouvert d'un revêtement non poreux empêchant l'échappement de la fragrance.

Diverses caractéristiques additionnelles, éventuellement combinées, sont indiquées ci-dessous.

Ainsi, de manière avantageuse, cette portion poreuse s'étend jusqu'à une extrémité libre de la tige de présentation, ce qui facilite le chargement en fragrance, typiquement par trempage dans de la fragrance liquide et chargement capillaire dans la masse poreuse de cette portion poreuse. Bien entendu, la totalité de la tige, sous la tête de préhension, peut être poreuse.

De manière également avantageuse, la portion poreuse de la tige est, dans la configuration de repos, à une distance non nulle d'un éventuel dépôt liquide de cette fragrance dans l'enceinte de confinement. Il n'est donc pas exclu qu'il y ait un dépôt liquide de fragrance au fond de l'enceinte de confinement, sous réserve que ce dépôt liquide ne soit pas au contact de la portion poreuse de la tige ; la présence d'un tel dépôt liquide peut avoir pour avantage de garantir que le volume interne de l'enceinte de confinement est en permanence saturé en fragrance, ce qui garantit que l'état de chargement de la portion poreuse en cette fragrance est sensiblement constant au cours du temps.

De manière également avantageuse, l'enceinte de confinement est conformée et orientée de telle sorte que, dans sa configuration de repos, la tige de présentation n'est en contact avec l'enceinte de confinement que dans la zone de col, la tige de présentation étant suspendue verticalement par sa tête de préhension. Cela évite la formation d'un point de goutte au contact entre la tige et l'intérieur de l'enceinte de confinement.

De manière également avantageuse, l'enceinte de confinement comporte une paroi interne qui longe avec jeu la surface externe de la tige de présentation sous la tête de préhension. Cela contribue à minimiser le volume de l'enceinte de confinement et donc la tendance de la fragrance à s'évaporer hors de la portion poreuse, lorsque la tige est en configuration de repos.

En effet, le fait que l'enceinte de confinement suit sensiblement le contour de la partie poreuse de la tige minimise les risques de déchargement de la fragrance dans cette enceinte lorsque la tige y est engagée ; la restitution de la fragrance se fait donc dans des conditions sensiblement constantes à chaque fois que cette tige est extraite, tandis qu'il y a peu (ou pas) de risques de chargement de l'atmosphère par la fragrance emmagasinée à l'état gazeux dans l'enceinte de confinement. Il faut bien comprendre que la notion de jeu est à prendre dans un sens large et qu'il peut s'agir de plusieurs millimètres en pratique.

De manière également avantageuse, l'enceinte a une section interne dont l'aire est au plus égale au double de l'aire de la section de la tige de présentation, ce qui garantit qu'il est facile d'extraire et d'engager la tige dans l'enceinte de confinement tout en minimisant le volume interne, et donc l'encombrement de l'enceinte de confinement.

En pratique, la section de la tige a avantageusement des dimensions comprises entre 0.5 et 5 centimètres, et la section interne de l'enceinte de confinement a avantageusement des dimensions comprises entre 1 et 10 centimètres. Des plages préférées pour ces dimensions sont de 1 à 3 centimètres pour les dimensions transverses de la section interne de la tige et de 1 à 5 centimètres pour les dimensions transverses de la section interne de l'enceinte de confinement.

Les tiges ont par exemple une longueur comprise entre 5 et 20 centimètres ; l'enceinte de confinement correspondante peut avoir, par rapport à la partie étroite de la tige, une longueur supérieure d'une valeur quelconque, par exemple comprise entre 1 et 5 centimètres. En fait, le fond de l'enceinte de confinement peut être renflé.

De manière avantageuse, la section de la tige de présentation et la section de l'enceinte de confinement sont, sous le col, sensiblement cylindriques ; en variante, la section de la tige de présentation et la section de l'enceinte de confinement sont, sous le col, sensiblement polygonales (par exemple carrée, rectangulaire, hexagonale, etc.). Ces sections peuvent être encore plus complexes en sorte, si cela est souhaité, que la tige ne puisse être engagée dans son enceinte de confinement que dans une seule configuration angulaire.

La partie poreuse de la tige peut être réalisée dans une grande variété de matériaux, dont le bois, des céramiques, etc.

De manière préférée, des dispositifs du type précité sont regroupés au sein d'un ensemble de conservation et de restitution (ou en abrégé un ensemble de présentation) de fragrances comportant une pluralité de dispositifs de conservation et de restitution (ou ensembles de présentation) dont les enceintes de confinement sont portées par un même support. Ces enceintes peuvent être engagées de manière amovible dans des orifices de ce support ou au contraire être solidaires de ce support, par exemple réalisées d'un seul tenant avec celui-ci. Les enceintes peuvent être disposées en réseau, par exemple en rangées et en colonnes ou en quinconce, notamment.

Pour des raisons esthétiques, les dispositifs de présentation ont, avantageusement, tous la même géométrie au sein d'un ensemble donné. Ils peuvent en outre avoir des dimensions identiques. En variante, les tiges et les enceintes de confinement des divers dispositifs de présentation ont au contraire, des dimensions ou des géométries spécifiques garantissant qu'une tige ne peut être engagée dans une autre enceinte que celle à laquelle elle est associée : cela garantit qu'il n'y aura pas de pollution d'une enceinte par une tige associée à un autre dispositif.

De manière avantageuse, l'ensemble de présentation comporte des éléments de marquage des tiges. Il peut s'agir de marques portées par les têtes de préhension (par exemple des points en creux ou en relief ou en couleur, excentrés par rapport à la tête de préhension) pouvant être orientées de différentes manières selon la position de la tige dans son enceinte, autour de sa direction longitudinale. En variante, ces éléments de marquage sont des éléments indépendants des tiges et des enceintes, tels que des bagues permettant de surélever une tête de préhension par rapport au col de l'enceinte de confinement correspondante.

L'invention propose en outre un procédé de conservation et de restitution de fragrances comportant les caractéristiques de la revendication 15.

Des objets, caractéristiques et avantages de l'invention ressortent de la description qui suit, donnée à titre d'exemple illustratif non limitatif, en référence au dessin annexé sur lequel :
- la figure 1 est une vue en coupe verticale d'un dispositif de présentation conforme à l'invention,
- la figure 2 est une vue de dessus de la tige de présentation du dispositif de la figure 1 faisant apparaître un premier type d'élément de marquage,
- la figure 3 est une vue en coupe de la partie supérieure du dispositif de la figure 1 avec un autre type de marquage, indépendant de la tige et de l'enceinte de confinement,
- la figure 4 est une vue de dessus de la tige de présentation du même dispositif avec une variante de réalisation de l'élément de marquage,
- la figure 5 est une vue en perspective partielle d'une batterie de dispositifs de présentation conformes à celui de la figure 1,
- la figure 6 en est une vue de dessus, montrant un usage possible des éléments de marquage visibles à la figure 2,
- la figure 7 est une vue partielle de la batterie de la figure 5 en coupe selon une ligne reliant plusieurs dispositifs, montrant un usage possible de l'élément de marquage de la figure 3,
- la figure 8 est une vue analogue à celle de la figure 7, montrant une variante de batterie,
- la figure 9 est une vue en coupe d'une variante de réalisation de la tige de présentation,
- la figure 10 est une vue en coupe d'une autre variante de réalisation de la tige de présentation,
- la figure 11 est une vue en coupe d'encore une autre variante de réalisation de la tige de présentation,
- la figure 12 est une vue en coupe d'encore une autre variante de réalisation de la tige de présentation,
- la figure 13 est une vue en coupe d'une variante d'un dispositif de présentation conforme à l'invention, et
- la figure 14 est une vue en coupe d'une autre variante de réalisation d'un dispositif de présentation conforme à l'invention.

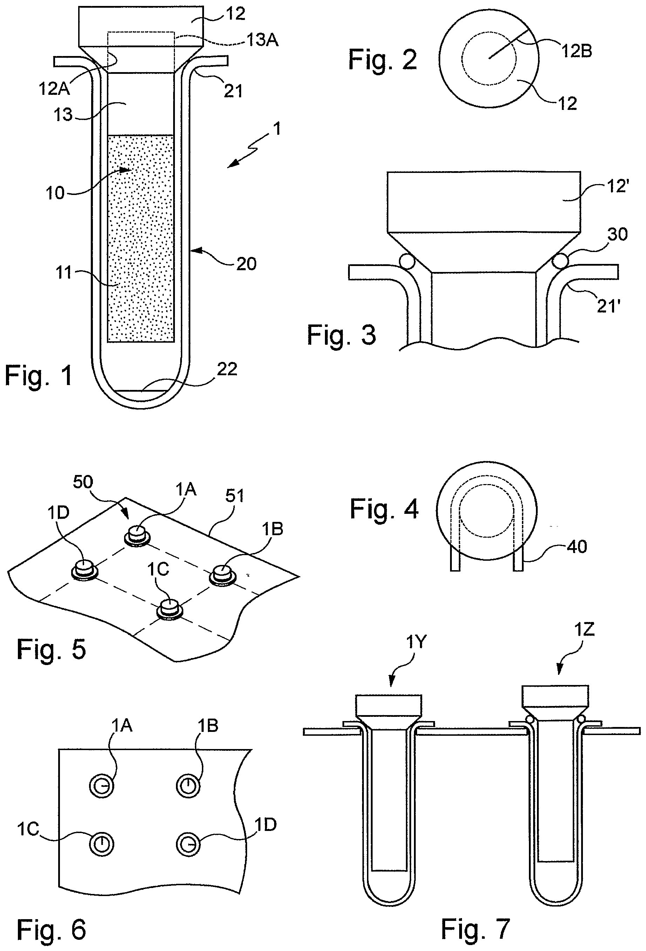

La figure 1 représente un dispositif 1 de présentation d'une fragrance donnée, comportant une tige de présentation 10 dans une enceinte de confinement 20. Ce dispositif de présentation assure à la fois la conservation et la restitution de la fragrance considérée.

La tige de présentation 10 comporte une portion poreuse 11 chargée en fragrance et au moins une autre portion 12 formant une tête de préhension.

L'enceinte de confinement 20 comporte un col 21 et la portion poreuse 11 de la tige de présentation est adaptée à traverser ce col 21 tandis que l'autre portion 12 est adaptée à obturer ce col dans une configuration de repos telle que la portion poreuse est, à l'intérieur de cette enceinte de confinement, dans une zone où la fragrance n'existe qu'à l'état gazeux. Cette configuration de repos est représentée à la figure 1 et on y voit, au fond de l'enceinte de confinement, un petit dépôt liquide 22, mais celui-ci est à une distance non nulle de la portion poreuse et plus généralement de la tige de présentation prise dans son ensemble. En fait un tel dépôt n'est pas recherché mais peut apparaître en fonction des conditions de chargement (c'est-à-dire des conditions d'imprégnation) en fragrance de la portion poreuse de la tige de présentation.

Dans l'exemple représenté, le col est évasé vers le haut. En variante non représentée, il est simplement formé, sans le moindre chanfrein, par le bord interne du rebord par lequel l'enceinte peut être retenue dans un support (voir la figure 5). De même, la tête peut être simplement formée par une portion de section constante supérieure à celle de la tige, raccordée à cette dernière par un épaulement annulaire transversal.

Pour des raisons de bon positionnement de la tête de préhension vis-à-vis du col, la tige de présentation présente avantageusement une portion tronconique à la liaison entre la tête et la portion basse de la tige (cette portion basse sous la tête est avantageusement de section constante sur toute sa longueur, mais peut aussi présenter une autre forme, par exemple effilée en direction de son extrémité libre).

L'extrémité inférieure de la tige proprement dite est ici constituée par une face inférieure mais, en variante non représentée, cette extrémité peut être arrondie (en d'autres termes, lorsque cette tige est de section constante, cette constance n'est plus satisfaite dans les derniers millimètres de cette tige).

Les matériaux constitutifs de la tige de présentation (au moins en dessous de la tête de préhension) et de l'enceinte de confinement sont dépourvus d'odeur propre, c'est-à-dire qu'ils sont neutres vis-à-vis de la fragrance et n'en modifient pas la perception.

On peut citer les matériaux suivants :
- des bois neutres tels que du bambou,
- de la pierre ponce,
- de la porcelaine,
- des mousses rigides,
- des éponges rigides.
- de la silice ou du silicate de calcium (du verre par exemple),
- des matières synthétiques telles que du polyméthylsiloxane,
- de la vermiculite,
- de la cellulose,
- des feutres,
- des filtres,
- des matières plastiques, etc.

En variante, au lieu d'être constitué d'un matériau rigide, la portion poreuse peut être constituée d'une enveloppe rigide poreuse ou munie d'une pluralité de perçages (en un matériau tel que ceux cités ci-dessus) contenant une matière molle adaptée à se charger en fragrance, une mousse ou une éponge par exemple.

De manière préférée, cette portion poreuse 11 s'étend jusqu'à une extrémité libre de la tige de présentation, ce qui est favorable du point de vue fabrication mais aussi du point de vue chargement (imprégnation) en fragrance.

En effet, la tige peut être en un matériau unique, pour la tige proprement dite et pour sa tête, et peut même être monobloc.

Toutefois, il est avantageux de fabriquer la tige de présentation en deux parties distinctes ; ainsi, on voit à la figure 1 que la tige de présentation 10 comporte une portion 13 de section constante et une portion qui forme la tête de préhension 12 et qui comporte un logement 12A dans laquelle est fixée l'extrémité supérieure 13A de la portion 13. Cette fixation peut être obtenue par tout moyen connu approprié, notamment par filetage, par collage ou par clipsage.

Lorsque la tige de présentation est réalisée en plusieurs parties, celles-ci peuvent être en des matériaux différents, choisis en fonction des rôles respectifs de ces parties ; c'est ainsi que le matériau de la portion de section constante est choisi en fonction de sa capacité à se charger en fragrance dans sa partie poreuse, tandis que celui de la tête de préhension peut être choisi selon des critères esthétiques.

Il peut bien sûr y avoir plus de deux parties. C'est ainsi que la portion de section constante peut être formée de deux parties distinctes dont l'une constitue la portion poreuse adaptée à se charger en fragrance et dont l'autre peut avoir une vocation plus esthétique. De même, la tête de préhension peut être formée d'un coeur recouvert d'un capot, par exemple en métal précieux (c'est ainsi que la tête peut être formée d'un seul tenant avec la tige proprement dite, donc en un matériau poreux, avec un éventuel revêtement non poreux). Si la partie de section constante est fixée de manière amovible, elle peut être remplacée, tout en conservant la tête en matériaux précieux.

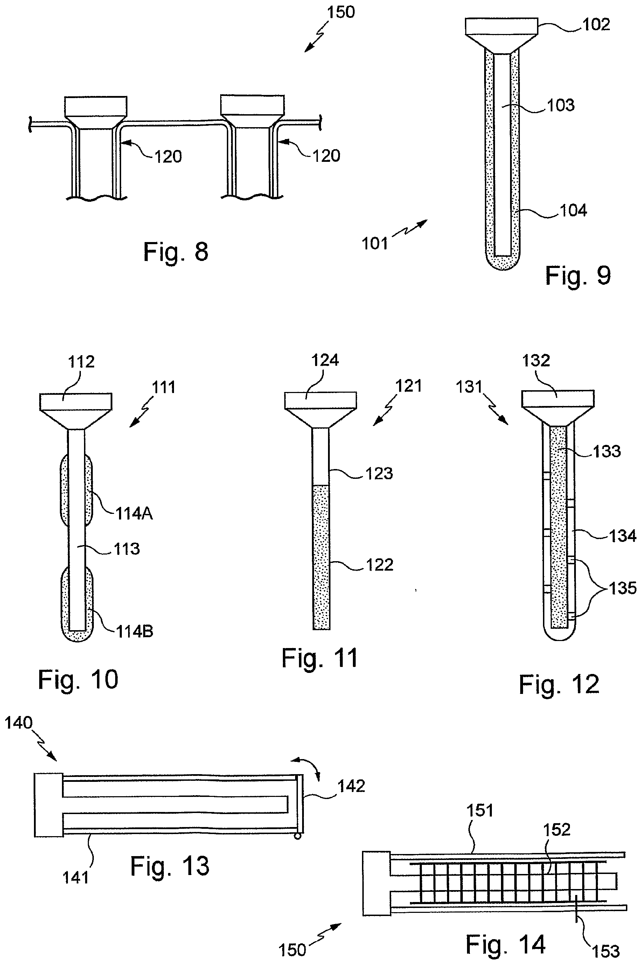

Diverses variantes de réalisation de la tige de présentation sont commentées ci-dessous à propos des figures 9 à 12.

Quant à l'enceinte de confinement, elle peut être réalisée en une grande variété de matériaux, compte tenu de ce que son rôle se limite à contenir, sans perversion, la tige chargée en fragrance. C'est ainsi qu'elle peut, notamment, être réalisée en verre, en métal (par exemple acier inoxydable) ou en une matière synthétique.

Ainsi que cela ressort de la figure 1, l'enceinte de confinement 20 est avantageusement conformée et orientée de telle sorte que, dans sa configuration de repos, la tige de présentation 10 n'est en contact avec l'enceinte de confinement que dans la zone de col, la tige de présentation étant suspendue verticalement par sa tête de préhension. De la sorte, il n'y a pas de contact entre la tige proprement dite et la paroi interne de l'enceinte de confinement, ce qui réduit les risques de formation de gouttes susceptibles de nuire à la présentation de la fragrance lors de l'extraction de la tige hors de l'enceinte de confinement.

Selon une autre caractéristique avantageuse de l'invention l'enceinte de confinement 20 comporte une paroi interne qui longe avec jeu la surface externe de la tige de présentation sous la tête de préhension (ce jeu peut être substantiel, par exemple de plusieurs millimètres). Cela explique que, pour éviter l'apparition d'une zone de contact entre la tige de présentation avec l'enceinte de confinement sous le col, l'enceinte est avantageusement disposée verticalement.

A titre d'ordre de grandeur de ce que peut représenter le jeu précité, éventuellement substantiel, on peut dire que l'enceinte a une section interne dont la section est au plus égale au double de la section de la tige de présentation, par contre le jeu est en pratique au moins égal à de l'ordre du millimètre.

Diverses formes peuvent être données à la partie basse de la tige de présentation (sous la tête de préhension), mais il est avantageux, pour des raisons de fabrication mais aussi de nature esthétique, que la forme choisie soit régulière, c'est-à-dire admette un axe ou des plans de symétrie. C'est ainsi que cette partie basse est avantageusement de section cylindrique (donc avec une section circulaire), mais peut aussi en variante être ovale, voire présenter un nombre de maxima de dimension supérieur à trois (par exemple selon une forme de trèfle).

En variante non représentée, cette partie basse peut aussi avoir une section de forme polygonale, par exemple carrée, rectangulaire, triangulaire ou hexagonale, notamment.

Cette partie basse peut aussi avoir une forme plus complexe, de manière à ce qu'elle ne puisse être engagée dans son enceinte de confinement que dans une configuration angulaire.

Dans l'exemple ici considéré d'une tige de section circulaire, l'enceinte de confinement a la forme générale d'une éprouvette cylindrique.

La tige a avantageusement une largeur transversale maximale comprise entre 0,5 et 5 centimètres (de préférence entre 1 et 3 centimètres) tandis que la section interne de l'enceinte de confinement a une largeur transversale maximale comprise entre 1 et 10 centimètres (de préférence entre 1 et 5 centimètres). La longueur de la tige (sous la tête) est avantageusement comprise entre 5 et 20 centimètres et l'enceinte peut comporter avoir son fond situé à une distance comprise entre 1 et 5 cm de l'extrémité de cette tige.

La tête de préhension peut avoir une grande variété de formes, à savoir des formes de bases telles que des formes cylindriques (comme dans l'exemple des figures 1 et 2), ovales ou polygonales, mais aussi des formes plus fantaisistes, telles qu'on peut en trouver pour des capuchons de flacons de parfum (forme de fleur, de flamme, de personnages, etc.).

A titre d'exemple, la tige a une partie étroite de 10 mm de diamètre, 120 mm de long avec une portion extrême poreuse de 30 mm de long, et une tête de 15 mm de diamètre et de 30 mm de long (cette tige a donc 150 mm de long). Quant à l'enceinte, elle a 124 mm de haut (y compris un rebord de 3,9 mm de haut), un diamètre externe de 14 mm (20 mm au niveau du rebord) avec un fond plat et une épaisseur de 0,85 mm.

La figure 2 représente la tête de préhension 12 de la figure 1 en vue de dessus. On observe la présence d'une marque 12B à l'écart de la zone centrale de cette tête, ici sensiblement orientée selon un rayon de cette tête. Cette marque peut être matérialisée par une gorge, une nervure ou par un ajout de couleur.

En variante non représentée, lorsque la tête a une forme asymétrique, notamment en cas de forme de fantaisie (voir ci-dessus), la marque peut être simplement constituée par un détail (relief ou creux) de cette forme de fantaisie (par exemple un pétale particulier dans le cas d'un motif de fleur).

On comprend qu'un tel élément de marquage permet un repérage de l'orientation de la tige de présentation par rapport à son enceinte de confinement. En effet ce sont des marques portées par les têtes de préhension qui peuvent être orientées de différentes manières selon la position de la tige dans son enceinte (il suffit pour ce faire que la section du col permette l'introduction de la tige de présentation selon au moins deux configurations possibles, par exemple dans le cas d'une section ovale ; ce qui précède n'est donc pas limité au cas où la partie basse de la tige est cylindrique).

Les figures 3 et 4 visualisent deux autres éléments de marquage (combinables avec le marquage précité), faisant intervenir un accessoire indépendant de la tige de présentation ainsi que de son enceinte de confinement.

Cet accessoire, noté 30 à la figure 3, est une bague annulaire adaptée à s'intercaler entre le col 21' de l'enceinte de confinement et la tête 12' de la tige correspondante, laquelle se trouve ainsi surélevée par rapport à sa position normale. Une démonstratrice peut ainsi repérer une fragrance, jugée intéressante par un client potentiel avant un choix ultérieur, par l'insertion d'une bague avant de remettre la tige correspondante dans son enceinte.

En variante l'élément de marquage peut être une pièce en U, notée 40 à la figure 4, qui peut être utilisée comme la bague précitée, mais qui est plus facile à enlever après que le client potentiel a finalisé son choix, sans avoir à retirer complètement la tige hors de son enceinte.

De manière avantageuse, un dispositif de présentation d'une fragrance donnée (tel que celui de la figure 1 ou un dispositif analogue) fait partie d'un ensemble de conservation et de restitution de fragrances (ou, plus simplement, de présentation de fragrances) comportant une pluralité de dispositifs de présentation dont les enceintes de confinement sont portées par un même support.

On voit ainsi à la figure 5 un réseau, ou batterie 50, de dispositifs de présentation, notés 1A, 1B, 1C, 1D distribués ici selon des rangées et des colonnes sur un même support noté 51. En variante non représentée, les dispositifs sont répartis en quinconce. Il peut, au contraire, y avoir une répartition irrégulière des dispositifs, selon un motif de fantaisie.

De manière préférée, les dispositifs de ce réseau ont tous la même géométrie. Toutefois, on peut prévoir, pour des raisons esthétiques notamment, de différencier les dispositifs selon les rangées, ou selon les colonnes, ou selon leur position par rapport à la périphérie, etc. On peut même prévoir que les tiges et enceintes de chaque dispositif de présentation ont des géométries ou des dimensions spécifiques garantissant qu'une tige ne peut être engagée que dans son enceinte de confinement ; en pratique, il peut suffire que les dispositifs de cet ensemble de la figure 5 soient différenciés vis-à-vis des dispositifs adjacents, de manière à éviter qu'une tige puisse être engagée dans une enceinte proche de l'enceinte du dispositif auquel appartient cette tige.

Lors d'une démonstration de fragrances, la démonstratrice a ainsi diverses fragrances à disposition, dans des conditions très similaires et l'éventuelle régularité du réseau des dispositifs peut contribuer à l'esthétique de cette démonstration.

De manière avantageuse, des éléments de marquage sont prévus, par exemple ceux décrits ci-dessus, pour permettre, après une première présentation de chaque fragrance, d'identifier celles entre lesquelles le choix final se fera.

On observe ainsi à la figure 6 que les dispositifs 1A et 1D ont leurs marques orientées vers la droite alors que les dispositifs 1B et 1C ont leurs marques orientées vers le haut. Suivant les conventions adoptées par la démonstratrice, cela peut signifier que les fragrances contenues dans les dispositifs 1B et 1C ont été préselectionnées pour l'étape suivante de choix, tandis que les fragrances contenues dans les dispositifs 1A et 1D n'ont pas été retenues.

Quant à la figure 7, elle montre côté à côte deux dispositifs 1Y et 1Z, dont l'un (ici 1Z) comporte une bague interposée sous la tête de préhension. Cela peut signifier que la fragrance contenue dans le dispositif 1Z a été retenu pour la suite du processus de choix, à la différence de la fragrance du dispositif 1Y.

Bien entendu, une grande variété de conventions peut être mises en oeuvre par la démonstratrice, en utilisant par exemple des bagues de plusieurs couleurs (pour caractériser le commentaire du client potentiel, notamment) ou pour procéder à plusieurs présélections (en intercalant plusieurs bagues sous une tête), etc.

Une démarche de présentation de fragrances présente typiquement les étapes suivantes :
- préparer une pluralité de dispositifs de présentation comportant chacun une tige de présentation dans une enceinte de confinement respective, cette enceinte de confinement comportant un col et cette tige de présentation comportant une portion poreuse adaptée à traverser ce col et une autre portion formant une tête de préhension, cette tige ayant une configuration de repos telle que la portion poreuse est à l'intérieur de cette enceinte de confinement, les matériaux constitutifs de la tige de présentation en dessous de la tête de préhension et de l'enceinte de confinement étant dépourvus d'odeur propre, la tête de préhension de chaque dispositif de présentation est adaptée à obturer ledit col dans ladite configuration de repos et la tige de chaque dispositif de présentation comporte, à l'opposé de la tête de préhension, une extrémité libre,
- charger chacune des tiges par une fragrance particulière et la mettre dans sa configuration de repos dans son enceinte de confinement, la portion poreuse étant dans une zone de l'enceinte de confinement respective où la fragrance n'existe qu'à l'état gazeux,
- extraire successivement une série de tiges de présentation hors de leurs enceintes de confinement en vue de la présentation de leur fragrance et, en fonction du choix d'un interlocuteur ou d'une interlocutrice, marquer cette tige comme étant sélectionnée, ou non, en l'engageant à nouveau en configuration de repos dans son enceinte de confinement, et
- extraire à nouveau hors de leurs enceintes de confinement, de manière successive, au moins certaines des tiges marquées comme étant sélectionnées.

De manière avantageuse, un dispositif de présentation correspond à une fragrance « neutre », c'est-à-dire une fragrance destinée à restaurer les facultés de l'utilisateur ou utilisatrice ; c'est ainsi qu'il peut être prévu, dans la batterie de dispositifs de présentation (on peut aussi parler d'orgue à parfum), un dispositif dont la tige est imprégnée d'un extrait de café ou de camphre, ou imprégnée d'une autre odeur non florale connue, pour que l'utilisateur (ou utilisatrice) puisse « nettoyer » son odorat avant de poursuivre ses évaluations des fragrances à sa disposition ; cela lui permet de sentir à nouveau d'autres fragrances et d'en percevoir les différences avec les précédentes fragrances testées.

Les figures 8 à 14 présentent diverses variantes de réalisation de la tige de présentation, d'un dispositif contenant une telle tige, ou d'un ensemble de présentation.

C'est ainsi que la figure 8 présente une variante de réalisation d'un ensemble de présentation de fragrances 150 qui se distingue de celui de la figure 7 par le fait que les enceintes de confinement 120 qui sont portées par un support commun, ne sont plus engagées de manière en pratique amovible dans des perçages de ce support, mais sont d'un seul tenant avec ce support.

Par ailleurs, diverses configurations peuvent être proposées pour la réalisation d'une tige de présentation en deux parties.

La tige 101 de la figure 9 comporte ainsi une tête 102 solidaire d'une âme centrale 103, en tout matériau approprié, métallique par exemple, revêtu d'un matériau poreux 104 : l'âme centrale constitue ainsi une armature capable d'assurer une bonne rigidité indépendamment de celle du matériau poreux, tandis que celui-ci peut n'être choisi qu'en fonction de ses caractéristiques d'imprégnation par les fragrances envisagées.

La figure 10 en représente une variante, repérée 111, selon laquelle le matériau poreux n'est engagé sur l'âme centrale 113 que sur une partie de la hauteur de celle-ci ; ce matériau est en pratique engagé au moins sur la partie basse de cette âme, mais il peut y avoir en outre une ou plusieurs autres portions à des niveaux plus proches de la tête de préhension. Il y a ici deux portions poreuses 114A et 114B (de moindre section que la tête 112).

La figure 11 est une variante de la figure 1, où la tige 121 est en deux parties, à savoir une partie basse 122 et une partie haute 123 ; toutefois, l'interface entre les parties haute et basse est ici à un niveau intermédiaire entre l'extrémité basse de la tige et la tête de préhension 124. La partie basse 122 correspond avantageusement à la portion poreuse 11 de la figure 1.

A l'inverse de ce qui est proposé par la figure 9, la partie centrale 133 de la tige 131 de la figure 12 peut être constituée par un matériau poreux, capable de s'imprégner en fragrance, tandis que cette partie centrale est entourée d'une gaine 134 en un autre matériau, par exemple non poreux, ici muni d'une pluralité de canaux 135 assurant la communication entre la partie centrale poreuse et l'extérieur. A titre d'exemple, ce matériau constitutif de la gaine est de l'émail, ce qui permet de multiples effets esthétiques.

Par ailleurs, selon un mode de réalisation qui ne fait pas partie de l'invention, la tige peut ne pas être obligatoirement sortie de son enceinte de confinement ; c'est ainsi que la figure 13 représente un dispositif 140 dont l'enceinte de confinement 141 est munie, à l'opposé du col dans lequel s'engage la tête de la tige de présentation, d'un orifice obturé par un couvercle amovible 142 ; il est ainsi possible de permettre la perception de la fragrance par simple action sur le dispositif d'ouverture/fermeture de l'enceinte, sans avoir à sortir la tige de cette enceinte.

Selon un autre mode de réalisation qui ne fait pas partie de l'invention, on peut même prévoir (voir le dispositif 150 de la figure 14) que l'enceinte 151 soit munie d'une grille 152, interposée entre la tige et l'enceinte, et que des mouvements d'extraction ou d'escamotage de la grille vis-à-vis de l'enceinte, par action sur un ergot 153 dont cette grille est muni et qui est accessible de l'extérieur (visible en bas à droite à la figure 14), favorisent la perception de la fragrance stockée dans l'enceinte considérée.

Plusieurs options sont possibles pour charger, en principe imprégner, une tige en une fragrance donnée.

C'est ainsi qu'une option consiste à prévoir, une fois par jour ou un autre rythme selon l'usage qui est fait des dispositifs, une opération spécifique de chargement, par exemple par trempage de la portion poreuse de la tige de présentation pendant des conditions, notamment de durée, conduisant à un chargement par capillarité approprié de la portion poreuse par la fragrance en cause. C'est une simple question d'étalonnage, à la portée de l'homme (ou femme) de métier que de définir les bonnes conditions pour un chargement en une fragrance donnée.

En variante, on peut prévoir que la tige est imprégnée lors de sa fabrication, ce qui correspond à des dispositifs de présentation de fragrances à usage limité (quoique bien supérieur aux bandes de papier actuelles).

Cela peut être intéressant pour la distribution d'échantillons à des consommatrices potentielles pour leur permettre des essais dans plusieurs conditions au cours de la journée.

En effet, un dispositif de présentation conforme à l'invention permet différents usages :
* permettre un choix parmi plusieurs fragrances possibles,
* distribuer des échantillons permettant plusieurs tests dans des conditions confortables,
* capter des fragrances pour en permettre ultérieurement l'analyse,
* etc.

On peut ajouter que le test peut se faire par contact de la partie poreuse avec la peau d'une cliente potentielle, ou simplement par inhalation du parfum qui se dégage de la portion poreuse.

Si on le souhaite, un joint, lié à la tête de préhension ou au col, peut être prévu entre ces deux éléments.

## Revendications

1. Dispositif de conservation et de restitution d'une fragrance donnée, comportant, dans une enceinte de confinement (20), une tige de présentation (10), cette enceinte de confinement comportant un col (21) et une zone où la fragrance n'existe qu'à l'état gazeux et cette tige de présentation comportant une portion poreuse (11) chargée en cette fragrance et adaptée à traverser ce col et une autre portion formant une tête de préhension (12), cette tige de présentation ayant une configuration de repos telle que la portion poreuse est située, à l'intérieur de cette enceinte de confinement, dans la zone où la fragrance n'existe qu'à l'état gazeux, ladite tige de présentation n'étant en contact avec la paroi de cette enceinte de confinement que dans la zone de col, les matériaux constitutifs de la tige de présentation en dessous de la tête de préhension et de l'enceinte de confinement étant dépourvus d'odeur propre, **caractérisé en ce que** la tête de préhension est adaptée à obturer ledit col dans ladite configuration de repos et cette tige de présentation comporte, à l'opposé de la tête de préhension, une extrémité libre en permettant des extractions successives hors de cette enceinte de confinement en vue de la présentation de cette fragrance.

2. Dispositif de conservation et de restitution selon la revendication 1, **caractérisé en ce que** cette portion poreuse s'étend jusqu'à ladite extrémité libre de la tige de présentation.

3. Dispositif de conservation et de restitution selon la revendication 1 ou la revendication 2, **caractérisé en ce que** la portion poreuse (11) de la tige est, dans la configuration de repos, à une distance non nulle d'un éventuel dépôt liquide de cette fragrance dans l'enceinte de confinement.

4. Dispositif de conservation et de restitution selon l'une quelconque des revendications 1 à 3, **caractérisé en ce que** l'enceinte de confinement est conformée et orientée de telle sorte que, dans sa configuration de repos, la tige de présentation (10) n'est en contact avec l'enceinte de confinement (20) que dans la zone de col, la tige de présentation étant suspendue verticalement par sa tête de préhension.

5. Dispositif de conservation et de restitution selon l'une quelconque des revendications 1 à 4, **caractérisé en ce que** l'enceinte de confinement (20) comporte une paroi interne qui longe avec jeu la surface externe de la tige de présentation sous la tête de préhension.

6. Dispositif de conservation et de restitution selon la revendication 5, **caractérisé en ce que** l'enceinte a une section interne dont la section est au plus égale au double de la section de la tige de présentation.

7. Dispositif de conservation et de restitution selon l'une quelconque des revendications 1 à 6, **caractérisé en ce que** la section de la tige de présentation et la section de l'enceinte de confinement sont, sous le col, sensiblement cylindriques.

8. Dispositif de conservation et de restitution selon l'une quelconque des revendications 1 à 7, **caractérisé en ce que** la section de la tige de présentation et la section de l'enceinte de confinement sont, sous le col, sensiblement polygonales.

9. Dispositif de conservation et de restitution selon l'une quelconque des revendications 1 à 8, **caractérisé en ce que** la tête de préhension est au moins en partie non poreuse.

10. Ensemble de conservation et de restitution de fragrances comportant une pluralité de dispositifs de conservation et de restitution (1A, 1B, 1C, 1D ; 1Y, 1Z) conformes à l'une quelconque des revendications 1 à 9, dont les enceintes de confinement sont portées par un même support (50).

11. Ensemble de conservation et de restitution selon la revendication 10, **caractérisé en ce que** les dispositifs de présentation ont tous la même géométrie.

12. Ensemble de conservation et de restitution selon la revendication 10 ou la revendication 11, **caractérisé en ce qu'**il comporte des éléments de marquage des tiges (12B, 30, 40).

13. Ensemble de conservation et de restitution selon la revendication 12, **caractérisé en ce que** les éléments de marquage sont des marques (12B) portées par les têtes de préhension pouvant être orientées de différentes manières selon la position de la tige dans son enceinte.

14. Ensemble de conservation et de restitution selon la revendication 12, **caractérisé en ce que** les éléments de marquage (30) sont des bagues permettant de surélever une tête de préhension par rapport au col de l'enceinte de confinement correspondante.

15. Procédé de conservation et de restitution de fragrances, comportant les étapes suivantes :
- préparer une pluralité de dispositifs de présentation (1A, 1B, 1C, 1D) comportant chacun une tige de présentation (10) dans une enceinte de confinement respective (20), cette enceinte de confinement comportant un col (21) et cette tige de présentation comportant une portion poreuse (11) adaptée à traverser ce col et une autre portion formant une tête de préhension (12), cette tige de présentation ayant une configuration de repos telle que la portion poreuse est à l'intérieur de cette enceinte de confinement, les matériaux constitutifs de la tige de présentation en dessous de la tête de préhension et de l'enceinte de confinement étant dépourvus d'odeur propre,
- charger chacune des tiges de présentation par une fragrance particulière et la mettre dans sa configuration de repos dans son enceinte de confinement, la portion poreuse étant dans une zone de l'enceinte de confinement respective où la fragrance n'existe qu'à l'état gazeux,
**caractérisé en ce que** la tête de préhension de chaque dispositif de présentation est adaptée à obturer ledit col dans ladite configuration de repos et la tige de chaque dispositif de présentation comporte, à l'opposé de la tête de préhension, une extrémité libre, et le procédé comporte les étapes suivantes :
- extraire successivement une série de tiges de présentation hors de leurs enceintes de confinement en vue de la présentation de leur fragrance et, en fonction du choix d'un interlocuteur ou d'une interlocutrice, marquer cette tige de présentation comme étant sélectionnée, ou non, en l'engageant à nouveau en configuration de repos dans son enceinte de confinement, et
- extraire à nouveau hors de leurs enceintes de confinement, de manière successive, au moins certaines des tiges de présentation marquées comme étant sélectionnées.

## Patentansprüche

1. Vorrichtung zum Aufbewahren und Ausgeben eines gegebenen Duftstoffs, die in einem Schutzbehälter (20) einen Präsentationsstab (10) enthält, wobei dieser Schutzbehälter einen Hals (21) und einen Bereich aufweist, in dem der Duftstoff nur in gasförmigem Zustand vorliegt, und wobei der Präsentationsstab einen porösen Abschnitt (11) enthält, der mit diesem Duftstoff beladen und dazu geeignet ist, sich durch den Hals zu erstrecken, sowie einen weiteren Abschnitt, der einen Greifkopf (12) bildet, wobei der Präsentationsstab eine Ruhekonfiguration derart hat, dass sich der poröse Abschnitt innerhalb des Schutzbehälters in dem Bereich befindet, wo der Duftstoff nur in gasförmigem Zustand vorliegt, wobei der Präsentationsstab nur im Halsbereich mit der Wand des Schutzbehälters in Kontakt steht, wobei die Materialien des Präsentationsstabs unterhalb des Greifkopfs und des Schutzbehälters keinen Eigengeruch haben,
**dadurch gekennzeichnet, dass**
der Greifkopf dazu geeignet ist, den Hals in der Ruhekonfiguration zu verschließen, und der Präsentationsstab dem Greifkopf entgegengesetzt ein freies Ende aufweist, das aufeinanderfolgende Extraktionen aus dem Schutzbehälter für die Präsentation des Duftstoffs ermöglicht.

2. Vorrichtung zum Aufbewahren und Ausgeben nach Anspruch 1, **dadurch gekennzeichnet, dass** der poröse Abschnitt sich bis zum freien Ende des Präsentationsstabs erstreckt.

3. Vorrichtung zum Aufbewahren und Ausgeben nach Anspruch 1 oder Anspruch 2, **dadurch gekennzeichnet, dass** der poröse Abschnitt (11) des Stabs in der Ruhekonfiguration in einem Abstand ungleich null von einer eventuellen flüssigen Ablagerung dieses Duftstoffs in dem Schutzbehälter liegt.

4. Vorrichtung zum Aufbewahren und Ausgeben nach einem der Ansprüche 1 bis 3, **dadurch gekennzeichnet, dass** der Schutzbehälter so geformt und ausgerichtet ist, dass in seiner Ruhekonfiguration der Präsentationsstab (10) nur in dem Halsbereich mit dem Schutzbehälter (20) in Kontakt steht, wobei der Präsentationsstab mit seinem Greifkopf vertikal aufgehängt ist.

5. Vorrichtung zum Aufbewahren und Ausgeben nach einem der Ansprüche 1 bis 4, **dadurch gekennzeichnet, dass** der Schutzbehälter (20) eine Innenwand enthält, die mit Spiel entlang der Außenfläche des Präsentationsstabs unter dem Greifkopf verläuft.

6. Vorrichtung zum Aufbewahren und Ausgeben nach Anspruch 5, **dadurch gekennzeichnet, dass** der Behälter einen Innenabschnitt aufweist, dessen Querschnitt höchstens gleich dem zweifachen Querschnitt des Präsentationsstabs ist.

7. Vorrichtung zum Aufbewahren und Ausgeben nach einem der Ansprüche 1 bis 6, **dadurch gekennzeichnet, dass** der Abschnitt des Präsentationsstabs und der Abschnitt des Schutzbehälters unter dem Hals im Wesentlichen zylindrisch sind.

8. Vorrichtung zum Aufbewahren und Ausgeben nach einem der Ansprüche 1 bis 7, **dadurch gekennzeichnet, dass** der Abschnitt des Präsentationsstabs und der Abschnitt des Schutzbehälters unter dem Hals im Wesentlichen polygonal sind.

9. Vorrichtung zum Aufbewahren und Ausgeben nach einem der Ansprüche 1 bis 8, **dadurch gekennzeichnet, dass** der Greifkopf zumindest teilweise nicht porös ist.

10. Einheit zum Aufbewahren und Ausgeben von Duftstoffen, enthaltend eine Mehrzahl von Vorrichtungen zum Aufbewahren und Ausgeben (1A, 1B, 1C, 1D; 1Y, 1Z) nach einem der Ansprüche 1 bis 9, deren Schutzbehälter von einem gleichen Träger (50) getragen werden.

11. Einheit zum Aufbewahren und Ausgeben nach Anspruch 10, **dadurch gekennzeichnet, dass** die Präsentationsvorrichtungen alle die gleiche Geometrie haben.

12. Einheit zum Aufbewahren und Ausgeben nach Anspruch 10 oder Anspruch 11, **dadurch gekennzeichnet, dass** sie Markierungselemente (12B, 30, 40) zum Markieren der Stäbe aufweist.

13. Einheit zum Aufbewahren und Ausgeben nach Anspruch 12, **dadurch gekennzeichnet, dass** die Markierungselemente Markierungen (12B) sind, die von den Greifköpfen getragen werden und auf verschiedene Arten je nach Position des Stabs in seinem Behälter ausgerichtet werden können.

14. Einheit zum Aufbewahren und Ausgeben nach Anspruch 12, **dadurch gekennzeichnet, dass** die Markierungselemente (30) Ringe sind, mit denen ein Greifkopf in Bezug auf den Hals des entsprechenden Schutzbehälters angehoben werden kann.

15. Verfahren zum Aufbewahren und Ausgeben von Duftstoffen, umfassend die nachfolgenden Schritte:
- Bereitstellen einer Mehrzahl von Präsentationsvorrichtungen (1A, 1B, 1C, 1D), die jeweils einen Präsentationsstab (10) in einem jeweiligen Schutzbehälter (20) enthalten, wobei dieser Schutzbehälter einen Hals (21) aufweist und dieser Präsentationsstab einen porösen Abschnitt (11) enthält, der dazu geeignet ist, sich durch den Hals zu erstrecken, sowie einen weiteren Abschnitt, der einen Greifkopf (12) bildet, wobei der Präsentationsstab eine Ruhekonfiguration derart hat, dass sich der poröse Abschnitt innerhalb des Schutzbehälters befindet, wobei die Bestandteilmaterialien des Präsentationsstabs unterhalb des Greifkopfs und des Schutzbehälters keinen Eigengeruch haben,
- Befüllen eines jeden der Präsentationsstäbe mit einem bestimmten Duftstoff und Verbringen in seine Ruhekonfiguration in seinem Schutzbehälter, wobei der poröse Abschnitt in einem Bereich des jeweiligen Schutzbehälters liegt, wo der Duftstoff nur in gasförmigem Zustand vorliegt,
**dadurch gekennzeichnet, dass**
der Greifkopf einer jeden Präsentationsvorrichtung dazu geeignet ist, den Hals in der Ruhekonfiguration zu verschließen, und der Stab einer jeden Präsentationsvorrichtung dem Greifkopf entgegengesetzt ein freies Ende aufweist, und
wobei das Verfahren die nachfolgenden Schritte umfasst:
- aufeinanderfolgende Extraktion einer Reihe von Präsentationsstäben aus deren Schutzbehältern heraus, um deren Duftstoff zu präsentieren, und in Abhängigkeit von der Wahl eines Ansprechpartners bzw. einer Ansprechpartnerin, Markieren dieses Präsentationsstabs als ausgewählt oder nicht ausgewählt, indem er erneut in die Ruhekonfiguration in seinem Schutzbehälter gebracht wird, und
- erneute aufeinanderfolgende Extraktion zumindest bestimmter der als ausgewählt markierten Präsentationsstäbe aus deren Schutzbehältern heraus.

## Claims

1. Device for storing and releasing a given fragrance, comprising a presentation stick (10) in a confining enclosure (20), this confining enclosure comprising a neck (21) and a zone in which the fragrance exists only in a gaseous state, and this presentation stick comprising a porous portion (11) laden with this fragrance and designed to pass through this neck and another portion forming a holding head (12), this presentation stick having a rest configuration such that the porous portion is, inside this confining enclosure, in the zone in which the fragrance exists only in the gaseous state, said presentation stick being in contact with the wall of this confining enclosure only in the neck region, the materials of which the presentation stick is made below the holding head and of which the confining enclosure is made having no odor of their own, **characterized in that** the holding head is designed to plug this neck in the rest configuration and this presentation stick comprises, opposite the holding head, a free end allowing extractions, in succession, out of this confining enclosure in order to exhibit this fragrance.

2. Storing and releasing device according to claim 1, **characterized in that** this porous portion extends as far as the free end of the presentation stick.

3. Storing and releasing device according to claim 1 or claim 2, **characterized in that** the porous portion (11) of the stick is, in the rest configuration, at a non-zero distance away from any potential liquid deposit of this fragrance inside the confining enclosure.

4. Storing and releasing device according to any one of claims 1 to 3, **characterized in that** the confining enclosure is shaped and oriented in such a way that, in its rest configuration, the presentation stick (10) is in contact with the confining enclosure (20) only in the neck region, the presentation stick being suspended vertically from its holding head.

5. Storing and releasing device according to any one of claims 1 to 4, **characterized in that** the confining enclosure (20) comprises an internal wall which runs, at a clearance, along the external surface of the presentation stick under the holding head.

6. Storing and releasing device according to claim 5, **characterized in that** the enclosure has an internal cross section the cross-sectional area of which is at most twice the cross-sectional area of the presentation stick.

7. Storing and releasing device according to any one of claims 1 to 6, **characterized in that** the cross section of the presentation stick and the cross section of the confining enclosure are, below the neck, substantially cylindrical.

8. Storing and releasing device according to any one of claims 1 to 7, **characterized in that** the cross section of the presentation stick and the cross section of the confining enclosure are, below the neck, substantially polygonal.

9. Storing and releasing device according to any one of claims 1 to 8, **characterized in that** the holding head is at least partially non-porous.

10. Set for storing and releasing fragrances comprising a plurality of storing and releasing devices (1A, 1B, 1C, 1D; 1Y, 1Z) according to any one of claims 1 to 9, the confining enclosures of which are carried by one and same support (50).

11. Set for storing and releasing fragrances according to claim 10, **characterized in that** the presentation devices all have the same geometry.

12. Set for storing and releasing fragrances according to claim 10 or claim 11, **characterized in that** it comprises stick-marking elements (12B, 30, 40).

13. Set for storing and releasing fragrances according to claim 12, **characterized in that** the marking elements are marks (12B) borne by the holding heads able to be oriented in different ways according to the position of the stick in its enclosure.

14. Set for storing and releasing fragrances according to claim 12, **characterized in that** the marking elements (30) are rings able to raise a holding head in relation to the neck of the corresponding confining enclosure.

15. Method for storing and releasing fragrances, comprising the following steps:
- preparing a plurality of presentation devices (1A, 1B, 1C, 1D) each comprising a presentation stick (10) in a respective confining enclosure (20), this confining enclosure having a neck (21) and this presentation stick having a porous portion (11) able to pass through this neck and another portion forming a holding head (12), this presentation stick having a rest configuration such that the porous portion is inside this confining enclosure, the materials of which the presentation stick below the holding head is made and of which the confining enclosure is made having no odor of their own,
- loading each of the presentation sticks with a particular fragrance and placing it in its rest configuration in its confining enclosure, the porous portion being in a region of the respective confining enclosure in which the fragrance exists only in the gaseous state,
**characterized in that** the holding head of each presentation device is designed to plug this neck in said rest configuration and the stick of each presentation device comprises, opposite the holding head, a free end, and the method comprises the following steps:
- extracting a series of presentation sticks in succession out of their confining enclosures in order to exhibit their fragrance, and, according to the choices expressed by the demonstrator's correspondent, marking this presentation stick as having been selected, or not selected, by fitting it back in its confining enclosure in the rest configuration, and
- once again, in succession, extracting, out of their confining enclosures, at least some of the presentation sticks, marked as having been selected.
